# EUROPÄISCHE PATENTSCHRIFT

(11) **EP 1 625 113 B1**
(45) Veröffentlichungstag und Bekanntmachung des Hinweises auf die Patenterteilung: **09.09.2020**
(21) Anmeldenummer: 04739104.0
(22) Anmeldetag: 23.04.2004
(51) Int. Cl.: C07C 319/08, B01J 23/30, B01J 37/20, B01J 37/28

(54) **KATALYSATOR ZUR HERSTELLUNG VON METHYLMERCAPTAN AUS METHANOL UND SCHWEFELWASSERSTOFF**
CATALYST FOR THE PRODUCTION OF METHYL MERCAPTAN FROM METHANOL AND HYDROGEN SULFIDE
CATALYSEUR DESTINE A LA PRODUCTION DE METHYLMERCAPTAN A PARTIR DE METHANOL ET D'ACIDE SULFHYDRIQUE

(30) Priorität: 30.04.2003 DE 10319739
(43) Veröffentlichungstag der Anmeldung: 15.02.2006
(73) Patentinhaber: BASF SE, 67056 Ludwigshafen am Rhein (DE)
(72) Erfinder: BRAND, Alexandra, 64291 Darmstadt (DE); QUASCHNING, Veronika, 68163 Mannheim (DE)
(74) Vertreter: BASF IP Association
(86) Internationale Anmeldenummer: PCT/EP2004/004344
(87) Internationale Veröffentlichungsnummer: WO 2004/096760

(56) Entgegenhaltungen:
- US-A- 5 847 223
- US-A- 5 852 219
- US-A- 5 874 630
- MASHKINA A V ET AL: "ACTIVITY OF TUNGSTATE CATALYSTS IN THE SYNTHESIS OF METHYL - MERCAPTANE FROM METHANOL AND HYDROGEN SULFIDE" REACTION KINETICS AND CATALYSIS LETTERS, ELSEVIER, AMSTERDAM, NL, Bd. 36, Nr. 1, 1988, Seiten 159-164, XP002063476 ISSN: 0133-1736 in der Anmeldung erwähnt

## Beschreibung

Die vorliegende Erfindung betrifft einen Katalysator zur Herstellung von Methylmercaptan aus Methanol und Schwefelwasserstoff.
Methylmercaptan ist ein wichtiges Zwischenprodukt für die Synthese von Methionin, zur Herstellung von Dimethylsulfoxid und Dimethylsulfon oder für die Synthese von Alkansulfonsäuren. Methylmercaptan wird heute überwiegend durch die Umsetzung von Schwefelwasserstoff und Methanol an einem Katalysator aus Aluminiumoxid in der Gasphase hergestellt. Die Synthese des Methylmercaptans erfolgt gewöhnlich bei Temperaturen zwischen 300 und 500°C und bei Drucken zwischen 1 und 25 bar.

Zur Erhöhung von Aktivität und Selektivität des Aluminiumoxidkatalysators wird dieser üblicherweise mit Alkaliwolframat dotiert (beispielsweise EP-A-832 878 oder US-A-585 2219). Mashkina et al. beschreibt, dass Katalysatoren, die saure Zentren an der Oberfläche enthalten, sehr aktiv sind, aber diese liefern jeweils gleiche Ausbeuten an Methylmercaptan und Dimethylsulfid. Katalysatoren, die starke basische Zentren aufweisen, sind weniger aktiv, weisen aber eine höhere Selektivität für Methylmercaptan auf (Mashkina et al., React. Kinet. Catal. Lett. 1987, 407-412). Die Aktivitäts- und Selektivitätssteigerung wird somit durch die Anwesenheit sowohl basischer als auch saurer Reaktionszentrum erklärt. Das Aluminiumoxid wird aber auch mit anderen Substanzen dotiert, beispielsweise mit Alkalicarbonat (US 5,847,223).

Eine Verbesserung von Aktivität und Selektivität erhält man auch durch Erhöhung des Molverhältnisses von Schwefelwasserstoff zu Alkohol. Üblicherweise werden Molverhältnisse zwischen 1 und 10 angewendet. Ein hohes Molverhältnis von 10 bis 3 bedeutet allerdings auch einen hohen Überschuss des Schwefelwasserstoffes im Reaktionsgasgemisch und somit die Notwendigkeit, große Gasmengen im Kreis zu führen.
US 2,685,605 betrifft ein Verfahren zur Herstellung von Methylmercaptan durch Umsetzung von Schwefelwasserstoff und Methanol an einem Katalysator, bevorzugt an einem Thorium/Bimsstein-Katalysator, wobei zusammen mit dem Schwefelwasserstoff und dem Methanol geringe Mengen Wasser in den Reaktor eingeleitet werden.
US 2,647,151 beschreibt die Herstellung von Alkylmercaptanen durch Umsetzung von Schwefelwasserstoff und Alkohol an einem Thorium/Bimsstein-Katalysator, worin die

Bildung der gewünschten Alkylmercaptane durch Unterdrückung der Bildung von als Nebenprodukten gebildeten organischen Sulfiden erhöht werden soll. Diese Selektivitätserhöhung wird dadurch erreicht, dass geringe Mengen Wasserstoff in den Reaktor geleitet werden.

DE-A-101 37 773 lehrt zusammen mit dem Alkohol und Schwefelwasserstoff bei laufender Umsetzung Sauerstoff in Gegenwart eines Katalysators aus Aluminiumoxid und Kaliumwolframat in den Reaktor einzuleiten. Die Sauerstoffzugabe verringert störende Ablagerungen auf dem Katalysator, und es erfolgt eine Regenerierung während des Verfahrens.

US 3,935,376 beschreibt ein Verfahren, dessen Verbesserung in einer Optimierung der Temperaturführung liegt, wobei Katalysatorqualitätsverluste und Nebenprodukte minimiert werden. Der Katalysator besteht aus Aluminiumoxid und einem Promotor. Es wird offenbart, dass es bei der Schaffung von mindestens drei Katalysatorzonen möglich ist, die Temperatur im gesamten Bereich bei der optimalen Temperatur zu halten. Die gesamte Menge an Schwefelwasserstoff wird in der ersten Katalysatorzone eingebracht, wobei die Methanolzugabe über die gesamten Katalysatorzonen verteilt werden kann. Das Verhältnis von Schwefelwasserstoff zu Methanol liegt zwischen 1.1 und 2.5.

In der EP-A-832 878 werden verbesserte Katalysatoren beschrieben, die dadurch erhältlich sind, dass der Kaliumwolframat Promoter in zwei Portionen auf dem aktiven Aluminiumoxid mittels einer speziellen Vorgehensweise abgeschieden wird. Desweiteren werden die Katalysatoren unter reaktionsähnlichen Bedingungen vorsulfidiert.

Mashkina et al. (React. Kinet. Catal. Lett. 1988,159-164) modifizierte die Einsatzform des Promotors, in dem hepta-, dodeca- und meta-Wolframat eingesetzt werden. Darüber hinaus wurden mit Kaliumcarbonat dotiertes Ammoniumwolframat, Kaliumwolframat mit geringem Säurezusatz und/oder mit Zugabe von Siliziumoxid eingesetzt. Das Ergebnis dieser umfangreichen Studie war, dass eine reine Kaliumwolframatdotierung ohne weitere Promotorenzugabe oder Behandlung im Bezug auf die Selektivität zu Methylmercaptan und die Reaktionsrate am geeignetesten war.

Gemäß SU 1316127, SU 1608923, RU 2056940 und WO 99/14172 wurde gefunden, dass man eine erhöhte Selektivität erhält bei der Verwendung von Aluminiumboratträgern oder durch Zugabe von Boroxiden zum Wolframatpromotor oder durch ein Promotorengemisch, dass Kaliumaluminat, amorphes Wolframoxid und Natriumoxid und/oder Boroxid enthält.

Die aus dem Stand der Technik bekannten Katalysatoren und Verfahrensweisen zur Herstellung von Methylmercaptan sind hinsichtlich der Selektivität und Aktivität bzw. wirtschaftlicher Verfahrensführung noch verbesserungswürdig.
Die Aufgabe der vorliegenden Erfindung war daher die Bereitstellung eines möglichst aktiven und selektiven Methylmercaptankatalysator, ohne dass eine mehrstufige Katalysatorherstellung, ein Einbringen teurer oder giftiger Aktivmassen oder eine besondere Reaktorfahrweise nötig ist. Desweiteren sollte zur Verminderung des benötigten Energieaufwandes das Verhältnis von Schwefelwasserstoff zu Alkohol nur wenig von 1 abweichen.

Diese Aufgabe wurde durch einen Katalysator gemäß Anspruch 1 gelöst. Weiterhin wurde gefunden, dass sich Methylmercaptan vorteilhaft unter Verwendung des erfindungsgemäßen Katalysator herstellen lässt. Als Alkaliwolframat wird Kaliumwolframat verwendet. Das Wolframat wird vorteilhaft mit 1 bis 20 Gew.-% bezogen auf die Gesamtmasse des Katalysators aufgetragen, bevorzugt mit 10 bis 16 Gew.-%.
Es werden bevorzugt Ammoniumsalze verwendet. Als Ammoniumsalze kommen besonders Sulfate, Phosphate, Sulfide, Wolframate, Molybdate, Sulfite, Peroxodisulfate, Phosphite, Hypophosphite, Halogenide und Carbonate in Betracht. Bevorzugt sind Sulfate, Phosphate, Sulfide, Wolframate, Molybdate, Sulfite, Peroxodisulfate, Phosphite und Hypophosphite. Besonders bevorzugt sind schwefel- oder phosphorhaltige Salze sowie Wolframatsalze. Gemische der Ammoniumsalze kommen ebenfalls in Betracht. Vorteilhaft werden 0,01 bis 15 Gew.-% Ammoniumsalze bezogen auf die Gesamtmasse des Katalysators aufgebracht, insbesondere 0,01 bis 10 Gew.-%.
Anstatt der Ammoniumsalze oder im Gemisch mit diesen Salzen ist auch insbesondere ein Einsatz von Protonensäuren ausgewählt aus der Gruppe bestehend aus Schwefelsäure, Phosphorsäure, Schwefliger Säure, Wolframsäure, Phosphorigen Säure und Hypo-phosphorigen Säure denkbar. Besonders bevorzugt sind schwefel- oder phosphorhaltige Protonensäuren.
Dieser Katalysator ist erfindungsgemäß dadurch erhältlich, in dem das aktivierte Aluminiumoxid mit dem Promotorengemisch enthaltend Alkaliwolframat und Ammoniumsalze getränkt wird oder in dem dieses Promotorengemisch auf den Aluminiumoxidkatalysator aufgesprüht wird. Das Tränken kann auf Wasseraufnahme, d.h. die Menge der Lösung entspricht dem Porenvolumen des Trägers, oder durch Überschusstränkung, d.h. das Lösungsvolumen ist größer als das Porenvolumen, erfolgen. Das Alkaliwolframat kann ebenso vor dem Aufbringen durch Umsetzung von Wolframatsalzen, wie beispielsweise Ammoniumwolframat, Ammonium-meta-wolframat, oder Ammonium-parawolframat, jeweils in Form der Hydrate, oder Wolframsäure mit beispielsweise Kalilauge oder Natronlauge hergestellt werden. Abschließend wird der Katalysator an Luft bzw. in Gegenwart von Sauerstoff calciniert, z. B. bei 400 bis 500°C.
Die Herstellung ist somit ein einfaches Einstufenverfahren. Gegebenenfalls kann das Aluminiumoxid vor dem Aufbringen der Promotoren für die Dauer von 1 bis 10 Stunden, bevorzugt 1 bis 5 Stunden, bei einer Temperatur von 300 bis 600°C, bevorzugt 400 bis 500°C calciniert werden.

Der erhaltenen Katalysator weist einen pH-Wert von 5 bis 9,7 auf. Die pH-Wert-Messung erfolgt, indem von dem zu untersuchenden Katalysator eine 10 %ige wässrige Suspension hergestellt wird. Diese Probe wird eine Minute geschüttelt, 5 Minuten stehen gelassen und anschließend wird von der Suspension der pH-Wert per Elektrode gemessen.
Dieser Katalysator führt gegenüber den aus dem Stand der Technik beschriebenen zu einer höheren Aktivität und Selektivität des fertigen Katalysators insbesondere bei niedrigem Molverhältnis von 3 bis 1 von Schwefelwasserstoff zu Methanol.

Als Aluminiumoxid für diesen Katalysator wird vorteilhaft sogenanntes aktives Aluminiumoxid eingesetzt. Dieses Material weist hohe spezifische Oberflächen zwischen etwa 10 und 400 m²/g auf und besteht hauptsächlich aus Oxiden der Übergangsreihe der kristallographischen Phasen des Aluminiumoxids (siehe zum Beispiel Ullmann's Enzyclopedia of Industrial Chemistry von 1985, Vol. A1, Seiten 561 - 562). Zu diesen Übergangsoxiden gehören χ-, κ-, γ-, δ-, η-, θ-Aluminiumoxid. Aktives Aluminiumoxid wird kommerziell für katalytische Anwendungen in verschiedenen Qualitäten und Lieferformen angeboten. Gut geeignet ist γ-Aluminiumoxid beispielsweise in granulierter oder stranggepresster Form. Vorteilhaft wird Aluminiumoxid mit Strangdurchmessern von 1 bis 5 mm verwendet. Die spezifischen Oberfläche liegt vorzugsweise bei 150 bis 400 m²/g. Das Gesamtporenvolumen liegt zwischen 0,3 und 1,0 ml/g. Die Schüttdichte befindet sich im Bereich von 300 bis 1000 g/l.
Der Katalysator kann gegebenenfalls vor dem Einsatz bei der Methylmercaptansynthese unter reaktionsähnlichen Bedingungen vorsulfidiert werden. Hierzu wird ein Schwefelwasserstoffstrom bei einer Temperatur von 200 bis 450°C und einem Druck von 1 bis 25 bar für die Dauer von 0,5 bis 100 Stunden über die Katalysatorpartikel geleitet.

Das molare Verhältnis von Schwefelwasserstoff zu Methanol beträgt bei der Methylmercaptansynthese im allgemeinen 1 zu 1 bis 10 zu 1, bevorzugt 1 zu 1 bis 2 zu 1. Dabei sind in den angegebenen molaren Verhältnissen sowohl die Anteile von frisch zugegebenem Schwefelwasserstoff und Methanol als auch die Anteile von recycliertem Schwefelwasserstoff und Methanol enthalten.

Der Katalysator wird im Reaktor vorteilhaft in Form von festen Partikel mit einem Durchmesser von 1 bis 5 mm, beispielsweise 4 mm, eingesetzt.

Der Reaktorfeed kann neben Schwefelwasserstoff und Alkohol Sauerstoff, Wasser oder Wasserstoff und des weiteren ein Inertgas oder eine Mischung von Inertgasen enthalten. Im allgemeinen enthält der Reaktorfeed 0 bis 30 mol, bevorzugt 5 bis 30 mol Inertgas pro mol Alkohol. Geeignete Inertgase sind z.B. Stickstoff, Methan, Ethan, Propan, Butan und/oder Kohlendioxid. Durch eine Sauerstoffzugabe können störende Ablagerungen auf dem Katalysator vermieden werden. Es kann somit eine Regenerierung des Katalysators während des Verfahrens erfolgen. Die Sauerstoffkonzentration kann in dem Verfahren im allgemeinen < 2,5 Gew.-% bezogen auf die Menge des Reaktorfeeds betragen und liegt somit weit unterhalb der Explosionsgrenzen. Bevorzugt kann die Sauerstoffkonzentration 10 ppm bis 0,5 Gew.-%, besonders bevorzugt von 10 ppm bis 500 ppm, betragen. Der Sauerstoff kann dem Reaktor in verschiedener Form zugeführt werden. Es ist möglich, reinen Sauerstoff einzusetzen, gegebenenfalls im Gemisch mit Inertgas. Überlicherweise wird der Sauerstoff in Form von Luft zugesetzt. Der Reaktorfeed kann weiterhin rückgeführte schwefelhaltige Komponenten enthalten, die vom erzeugten Methylmercaptan abgetrennt wurden (wie beispielsweise in DE-A-1768826 beschrieben).

Das erfindungsgemäße Verfahren zur Herstellung von Methylmercaptanen durch Umsetzung von Schwefelwasserstoff und Methanol an einem Katalysator wird im allgemeinen als Gasphasenreaktion in einem Rohrreaktor durchgeführt. Es können auch mehrere Rohrreaktoren hintereinander geschaltet verwendet werden. Im allgemeinen werden Methanol und Schwefelwasserstoff auf eine Temperatur geheizt, die hoch genug ist, dass sowohl Methanol als auch Methylmercaptan in der Dampfphase vorliegen, die jedoch unter der Zersetzungstemperatur des Methylmercaptans liegt. Im allgemeinen wird das erfindungsgemäße Verfahren bei Temperaturen zwischen 250 und 500°C, bevorzugt zwischen 300 und 450°C durchgeführt. Die genaue Reaktionstemperatur ist unter anderem abhängig vom Reaktionsdruck und dem eingesetzten Katalysator.

Das erfindungsgemäße Verfahren wird im allgemeinen bei einem Druck von 1 bis 25 bar durchgeführt. Selbstverständlich wird der Druck nicht so hoch gewählt, dass der Reaktorfeed bzw. das Methylmercaptan kondensiert. Bevorzugt beträgt der Druck in dem erfindungsgemäßen Verfahren 1 bis 10 bar. Aus Gründen eines verminderten Emissionsrisikos kann er auf 1 bis 3 bar eingestellt werden, bevorzugt annährend drucklos.

Das erfindungsgemäße Verfahren wird im allgemeinen kontinuierlich durchgeführt. Die Aufarbeitung des erhaltenen Methylmercaptans erfolgt dabei nach dem Fachmann bekannten Methoden.

Die WHSV (Weight hourly space velocity = Gewichte Edukte / Gewicht Katalysator pro Rohr und Stunde) beträgt im allgemeinen 0,1 bis 10 h⁻¹, bevorzugt 0,1 bis 5 h⁻¹, besonders bevorzugt 0,5 bis 2 h⁻¹.

Der Umsatz beträgt in dem erfindungsgemäßen Verfahren im allgemeinen 80 bis 100%, bevorzugt 95 bis 100%, bezogen auf die Menge der im molaren Unterschuss eingesetzten Komponente (also bezogen auf Methanol in der Methylmercaptansynthese). Mit dem erfindungsgemäßen Verfahren kann eine Methylmercaptan-Selektivität von 80 bis 100% erreicht werden.

### Beispiele:

### Beispiel 1: Herstellung des Vergleichskatalysator (14 Gew.-% K₂WO₄ auf γ-Al₂O₃)

203 g Ammonium-meta-wolframat-hydrat wurden in 0,385 l Wasser gelöst, dann wurden 182 g einer 48 %igen Kalilauge zugegeben und anschließend auf ein Volumen entsprechend der Wasseraufnahme des Trägers aufgefüllt. Diese 1,048 l Imprägnierlösung wurde gleichmäßig auf 1,588 kg γ-Al₂O₃ Stränge aufgesprüht. Abschließend wurde der Katalysator 2 h im Umluftofen bei 450°C calciniert.

Herstellung des erfindungsgemäßen Katalysators aus Aluminiumoxid, Kaliumwolframat und Ammoniumsalzen

### Beispiel 2: Ammoniumphosphat

72,3 g Ammonium-meta-wolframat-hydrat wurden in 0,150 l Wasser gelöst, dann wurden 29,7 g Kaliumhydroxid und 88,3 g Ammoniumphosphat-Trihydrat zugegeben. Diese Lösung wurde auf ein der Wasseraufnahme des Trägers entsprechendes Volumen aufgefüllt. Die Tränklösung wurde gleichmäßig auf 500 g γ-Al₂O₃ Stränge aufgegeben und der Katalysator wurde abschließend 2 h im Umluftofen bei 450°C calciniert.

### Beispiel 3: Ammoniumsulfat

Analog zu Beispiel 2 wurde eine wässrige Lösung aus Ammonium-meta-wolframat-hydrat, Kaliumhydroxid und 30,9 g Ammoniumsulfat hergestellt, auf 500 g γ-Al₂O₃ Stränge aufgegeben und der Katalysator abschließend 2 h im Umluftofen bei 450°C calciniert.

### Beispiel 4: Ammoniumsulfid

Analog zu Beispiel 2 wurde eine wässrige Lösung aus Ammonium-meta-wolframat-hydrat, Kaliumhydroxid und 36,1 g Ammoniumsulfid hergestellt, auf 500 g γ-Al₂O₃ Stränge aufgegeben und der Katalysator abschließend 2 h im Umluftofen bei 450°C calciniert.

### Charakterisierung der Katalysatoren

| Katalysator | Promotoren | | S_{BET} [m²[g] | pH-Wert | Chem. Analyse [Gew.-%] |
|---|---|---|---|---|---|
| | Art | Menge [Gew.-%] | | | |
| Vgl.-Bsp. 1 | - | - | 221 | 9,8 | - |
| Bsp. 2 | Ammoniumphosphat | 5 % P₂O₅ | 162 | 7,9 | P: 2,2 |
| Bsp. 3 | Ammoniumsulfat | 5% (NH₄)₂SO₄ | 190 | 6,0 | S: 1,1 |
| Bsp. 4 | Ammoniumsulfid | 3 % (NH₄)₂S | 189 | 8,2 | S: 0,4 |

pH-Wert-Messung: Von dem zu untersuchenden Katalysator wurde eine 10 %ige wässrige Suspension hergestellt. Die Probe wurde eine Minute geschüttelt, 5 Minuten stehen gelassen und anschließend wurde von der Suspension der pH-Wert per Elektrode gemessen.
S_{BET}-Messung: DIN 66131

### Performancetest:

Der MeSH-Reaktor (600 mm lang, 25 mm Durchmesser) wurde mit 280 g Katalysatorsträngen gefüllt. Bei einer Temperatur von 390°C (Reaktormitte) und einem Druck von 1,1 bar wurden 48 g/h (1,5 mol) gasförmiges Methanol und 64 g/h (1,9 mol) Schwefelwasserstoff in den Rohrreaktor eingespeist. Die Gaszusammensetzung wurde gaschromatographisch bestimmt.

| Beispiel | Hot-spot-Temp. [°C] | Zersetzungsprodukte [GC-FI.%] | Selektivität [%] | MeOH-Umsatz [%] | MeSH-Ausbeute [%] |
|---|---|---|---|---|---|
| Vgl.-Bsp. 1 | 432 | 1,10 | 83,8 | 97,7 | 81,9 |
| Bsp.2 | 440 | 1,78 | 89 | 99 | 89 |
| Bsp. 3 | 440 | 1,44 | 89 | 97 | 87 |
| Bsp. 4 | 443 | 1,38 | 88 | 97 | 86 |

## Patentansprüche

1. Katalysator für die Synthese von Methylmercaptan, erhältlich aus Aluminiumoxid, Alkaliwolframat und mindestens einer weiteren Komponente ausgewählt aus den Gruppen der Ammoniumsalze und der Protonensäuren Schwefelsäure, Phosphorsäure, Schwefligen Säure, Wolframsäure, Phosphorigen Säure, Hypophosphorigen Säure, oder deren Gemische, **dadurch gekennzeichnet, dass** der pH-Wert des Katalysators im Bereich von 5.0 bis 9.7 liegt, wobei die pH-Wert-Messung erfolgt, indem von dem zu untersuchenden Katalysator eine 10 %ige wässrige Suspension hergestellt, diese Probe eine Minute geschüttelt, 5 Minuten stehen gelassen und anschließend von der Suspension der pH-Wert per Elektrode gemessen wird, und wobei als Alkaliwolframat ein Kaliumwolframat verwendet wird, und wobei der Katalysator in Gegenwart von Sauerstoff calciniert wird.

2. Katalysator nach Anspruch 1, erhältlich aus Aluminiumoxid, Alkaliwolframat und mindestens einem Ammoniumsalz.

3. Katalysator nach Anspruch 1 oder 2, wobei als Ammoniumsalze Sulfate, Phosphate, Sulfide, Wolframate, Molybdate, Sulfite, Peroxodisulfate, Phosphite und Hypophosphite verwendet werden.

4. Katalysator nach Anspruch 1 oder 2, wobei als Ammoniumsalze schwefel- oder phosphorhaltige Salze oder Wolframatsalze verwendet werden.

5. Katalysator nach Anspruch 1 oder 2, wobei Alkaliwolframate mit 10 bis 16 Gew.-% bezogen auf die Gesamtmasse des Katalysators aufgetragen werden.

6. Katalysator nach Anspruch 1 oder 2, wobei Ammoniumsalze mit 0,01 bis 15 Gew.-% bezogen auf die Gesamtmasse des Katalysators aufgetragen werden.

7. Verfahren zur Herstellung von Methylmercaptanen durch Umsetzung von Methanol mit Schwefelwasserstoff, **dadurch gekennzeichnet, dass** man einen Katalysator nach den Ansprüchen 1 bis 6 verwendet.

8. Verfahren nach Anspruch 7, **dadurch gekennzeichnet, dass** bei der Herstellung von Methylmercaptanen Schwefelwasserstoff und Methanol in einem molaren Verhältnis von 1 zu 1 bis 2 zu 1 eingesetzt werden.

## Claims

1. A catalyst for the synthesis of methyl mercaptan, obtainable from aluminum oxide, an alkali metal tungstate and at least one further component selected from the groups of ammonium salts and of the protic acids sulfuric acid, phosphoric acid, sulfurous acid, tungstic acid, phosphorous acid, hypophosphorous acid or mixtures thereof, wherein the pH of the catalyst is in the range from 5.0 to 9.7, where the pH measured is carried out by producing a 10% strength aqueous suspension from the catalyst to be examined, shaking this sample for one minute, allowing it to stand for 5 minutes and subsequently measuring the pH of the suspension by means of an electrode and where a potassium tungstate is used as alkali metal tungstate and where the catalyst is calcined in the presence of oxygen.

2. The catalyst according to claim 1 which is obtainable from aluminum oxide, alkali metal tungstate and at least one ammonium salt.

3. The catalyst according to claim 1 or 2, wherein sulfates, phosphates, sulfides, tungstates, molybdates, sulfites, peroxodisulfates, phosphites and hypophosphites are used as ammonium salts.

4. The catalyst according to claim 1 or 2, wherein the sulfur- or phosphorus-comprising salts or tungstate salts are used as ammonium salts.

5. The catalyst according to claim 1 or 2, wherein alkali metal tungstates are applied in an amount of from 10 to 16% by weight, based on the total mass of the catalyst.

6. The catalyst according to claim 1 or 2, wherein ammonium salts are applied in an amount of from 0.01 to 15% by weight, based on the total mass of the catalyst.

7. A process for preparing methyl mercaptans by reacting methanol with hydrogen sulfide, wherein a catalyst according to any of claims 1 to 6 is used.

8. The process according to claim 7, wherein hydrogen sulfide and methanol are used in a molar ratio of from 1:1 to 2:1 in the preparation of methyl mercaptans.

## Revendications

1. Catalyseur pour la synthèse de méthylmercaptan, pouvant être obtenu à partir d'oxyde d'aluminium, de tungstate d'alcali et d'au moins un composant supplémentaire choisi dans les groupes des sels d'ammonium et des acides protoniques - acide sulfurique, acide phosphorique, acide sulfureux, acide tungstique, acide phosphoreux, acide hypophosphoreux - ou leurs mélanges, **caractérisé en ce que** la valeur de pH du catalyseur se situe dans la plage de 5,0 à 9,7, la mesure de la valeur de pH étant réalisée par le fait qu'une suspension aqueuse à 10 % est préparée à partir du catalyseur à examiner, cet échantillon est agité une minute, laissé à reposer 5 minutes et ensuite la valeur de pH de la suspension est mesurée par des électrodes, et un tungstate de potassium étant utilisé en tant que tungstate d'alcali, et le catalyseur étant calciné en présence d'oxygène.

2. Catalyseur selon la revendication 1, pouvant être obtenu à partir d'oxyde d'aluminium, de tungstate d'alcali et d'au moins un sel d'ammonium.

3. Catalyseur selon la revendication 1 ou 2, des sulfates, des phosphates, des sulfures, des tungstates, des molybdates, des sulfites, des peroxodisulfates, des phosphites et des hypophosphites étant utilisés en tant que sels d'ammonium.

4. Catalyseur selon la revendication 1 ou 2, des sels contenant du soufre ou contenant du phosphore ou des sels de tungstate étant utilisés en tant que sels d'ammonium.

5. Catalyseur selon la revendication 1 ou 2, des tungstates d'alcali étant déposés à raison de 10 à 16 % en poids par rapport à la masse totale du catalyseur.

6. Catalyseur selon la revendication 1 ou 2, des sels d'ammonium étant déposés à raison de 0,01 à 15 % en poids par rapport à la masse totale du catalyseur.

7. Procédé pour la préparation de méthylmercaptans par transformation de méthanol avec du sulfure d'hydrogène, **caractérisé en ce qu'**on utilise un catalyseur selon les revendications 1 à 6.

8. Procédé selon la revendication 7, **caractérisé en ce que** le sulfure d'hydrogène et le méthanol sont utilisés en un rapport molaire entre 1 à 1 et 2 à 1 pour la préparation de méthylmercaptans.
